(19) Europäisches Patentamt

European Patent Office

Office européen des brevets

(11) Publication number: **0 161 834**
**B1**

(12) **EUROPEAN PATENT SPECIFICATION**

(45) Date of publication of the patent specification:
07.01.88

(51) Int. Cl.⁴: **G 02 B 23/08**, A 61 B 1/00

(21) Application number: 85302816.5

(22) Date of filing: 23.04.85

(54) Objective head for fibrescopes.

(30) Priority: 04.05.84 US 607035

(43) Date of publication of application:
21.11.85 Bulletin 85/47

(45) Publication of the grant of the patent:
07.01.88 Bulletin 88/1

(84) Designated Contracting States:
DE FR GB

(56) References cited:
DE - A - 2 805 451
DE - A - 2 843 553
US - A - 4 245 624

PATENTS ABSTRACTS OF JAPAN, vol. 6, no. 8 (P-98)
[886], 19th January 1982

(73) Proprietor: Warner-Lambert Technologies, Inc.,
122 Charlton Street, Southbridge
Massachusetts 01550 (US)

(72) Inventor: Carpenter, George J., 42 Edwards Street,
Southbridge Massachusetts 01550 (US)

(74) Representative: Coxon, Philip et al, Eric Potter &
Clarkson 14 Oxford Street, Nottingham NG1 5BP (GB)

## Description

The present invention relates to fibrescopes useful in medical, veterinary and industrial applications using bundles of optical fibres to illuminate and to transmit images of remotely located objects.

Because of the nature of glass fibres (numerical aperture), it is frequently necessary to utilize a lens or lens system at the distal or objective end of light guide and imaging fibre optic bundles.

In prior art fibrescopes the various light guide and imaging bundles are received and anchored within a metallic element known as an objective head. The metallic head is machined to provide recesses and appropriate through bores to accommodate fibre bundles and to provide outlets for the usual utility channels such as for air, water, suction and biopsy instruments.

The required lenses, usually of glass, are individually manufactured at relatively high cost because of their small size; they are usually 1 to 2 mm in diameter by .75 to 1.0 mm thick. These small glass objects are cemented into machined counterbores in the metallic head.

A representative example of the prior art is disclosed in U.S. Patent No. 4 266 534 issued on May 12, 1981. In this disclosure a variety of lenses 6 are shown cemented to machined counterbores in a metallic objective head.

Such prior art heads suffer from a number of problems. One problem is the risk and the dangerous consequence of a lens falling out of its setting and detaching from the instrument during a critical medical, veterinary or industrial procedure. Another problem is the expense of manufacture.

A further problem is ability of the prior art head to conduct electricity. Since various electrical power sources are frequently associated with modern fibrescopes, it has become necessary to provide electrical earths to insure against injury resulting from random or stray currents transmitted through a metallic head.

It is an object of the present invention to provide an improved objective head structure which avoids the above disadvantages.

According to one aspect of the present invention we provide an objective head for an image-transmitting fibrescope useful in medical, veterinary and industrial applications of the type having at the distal or objective end an objective head having recesses for receiving image and lighting fibre optic bundles and outlets for utility channels such as for air, irrigation, suction and biopsy instruments characterised in that it comprises an optically clear plastic structure having at least one recess for receiving and anchoring at least one light guide fibre optic bundle and/or at least one imaging fibre optic bundle and at least one lens associated with the or each recess and the or each fibre optic bundle received therein, the lenses and the head being an integral single piece structure.

The invention includes fibrescopes incorporating an objective head as defined above.

According to another aspect of the present invention we provide a method of producing an objective head for a fibrescope, said head having at least one recess for receiving a fibre optic bundle comprising the steps of:

providing a mould in the desired configuration of the head,

placing a core in the mould so that a bottom wall of said recess defines a lens shape,

moulding the head using optically clear plastic material, and

removing the core from the moulded head thereby creating a moulded plastic objective head with the lens and the head being an integral single piece structure.

Reference is now made to the accompanying drawings, in which:

Figure 1 is a schematic representation of part of a prior art objective head;

Figure 2 is a schematic view of an end face of an objective head of the present invention;

Figure 3 is a sectional view along the line 3-3 of Figure 2; and

Figure 4 is a sectional view along the line 4-4 of Figure 2.

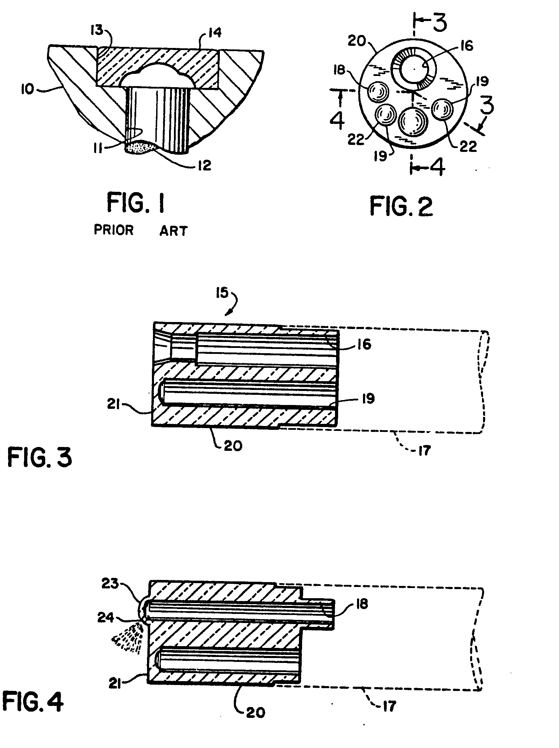

Referring to Figure 1 of the drawings, which is a fragmentary view of a prior art device, a machined metallic sleeve 10 is formed with a bore 11 for receiving a fibre optic bundle 12. A machined recess or counterbore 13 provides a receptacle or setting for a lens 14 fabricated as a separate piece-structure and cemented in place.

In contrast an objective head according to the present invention is shown in Figures 2, 3 and 4. A generally cylindrical unitary objective head indicated generally by the reference numeral 15 is moulded or cast from an optically clear plastic material, preferably a polycarbonate resin. Other suitable resins include acrylics and urethanes.

The objective head 15 includes a body 20 including a through bore 16 which communicates with a channel within a flexible shaft 17 of a fibrescope in well-known fashion. The bore 16 provides a conduit which can, for example, be used for a biopsy instrument and can be switched selectively to a source of suction. A bore 18 can communicate with a source of water and air in a usual and customary manner.

The bores 19-19 receive and anchor fibre optic bundles (not shown) serving as light guides providing illumination at the face 21 of the head 15, the light being spread over the field of view of the imaging system by light guide lenses 22-22.

The lenses 22-22 are moulded or cast integrally with the body 20 so at to form a single piece-structure. These lenses 22-22 also serve as a seal protecting the light guide fibre optic bundles.

As can be seen in Figure 4 the bore 18 has a rounded protuberance or projection 23 extending slightly beyond the face of the objective head. This protuberance 23 affords a means for directing air or water in a predetermined direction across the face 21 of the objective head. Air or water, as the case may be, is directed in a predetermined direction by forming one or more orifices such as orifice 24 (Figure 4) in an appropriate location in the protuberance 23.

The protuberance 23 is moulded integrally and forms a single piece-structure with the lenses 22-22 and the body 20.

The image fibre optic bundle received in channel 15 (Figure 4) is also provided with an integral lens 25. Thus all fibre optic bundles are provided with a safe, cement-free lens and a liquid-tight seal protecting the fibre optic bundles from moisture arising from a medical, veterinary or industrial procedure or from sterilization.

The objective head 15 may be formed or fabricated by preparing a mould cavity in the desired configuration, inserting in the mould a mould core to define the lens shape, injecting optically clear plastic material into the mould and after the plastic is set removing the core to provide a unitary objective head with «built-in» lens.

If desired, the exterior of the head may be coated with a film of scratch-resistant material such as Permalite or Duralite (both are trade names for organic silanes) to preserve the clarity of the plastic.

**Claims**

1. An objective head for an image-transmitting fibrescope useful in medical, veterinary and industrial applications of the type having at the distal or objective end an objective head having recesses for receiving image and lighting fibre optic bundles and outles for utility channels such as for air, irrigation, suction and biopsy instruments characterised in that it comprises an optically clear plastic structure having at least one recess (19) for receiving and anchoring at least one light guide fibre optic bundle and/or at least one imaging fibre optic bundle and at least one lens (22) associated with the or each recess and the or each fibre optic bundle received therein, the lenses and the head being an integral single piece structure.

2. An objective head according to Claim 1 in which the head is formed with at least one utility outlet (16) operable to communicate with a utility channel in a fibrescope.

3. An objective head according to Claim 2 in which one utility channel outlet defines a bore (18) open at one end and substantially closed at the opposite end by a protuberance (23) which is an integral part of the head, said protuberance having an orifice (24) located in a predetermined position effective to direct fluid passing through said bore across the face of said head.

4. An objective head according to any of Claims 1 to 3 moulded from polycarbonate resin.

5. An objective head according to any of Claims 1 to 4 having an exterior coating or transparent optically clear scratch-resistant material.

6. An image-transmitting fibrescope useful in medical, veterinary and industrial applications of the type having at the distal or objective end an objective head having recesses for receiving image and lighting fibre optic bundles and outlets for utility channels such as for air, irrigation, suction and biopsy instruments, characterised in that it has an objective head as claimed in any of Claims 1 to 5.

7. A method of producing an objective head for a fibrescope, said head having at least one recess for receiving a fibre optic bundle comprising the steps of:

providing a mould in the desired configuration of the head,

placing a core in the mould so that a bottom wall of said recess defines a lens shape,

moulding the head using optically clear plastic material, and

removing the core from the moulded head thereby creating a moulded plastic objective head with the lens and the head being an integral single piece structure.

8. A method according to Claim 7 including the additional step of coating the exterior of the head with a skin of film of transparent optically clear scratch-resistant material.

**Patentansprüche**

1. Objektivkopf für bildübertragende Faseroptiken in der Medizin, der Veterinärmedizin und in industriellen Anwendungen, welche am distalen Ende bzw. am Objektivende einen Objektivkopf aufweisen, der mit Ausnehmungen zur Aufnahme von Bild- und Lichtleiterfaserbündeln sowie mit Durchlässen für Hilfskanäle für Luft, Befeuchtung, Saug- und Biopsieinstrumenten versehen ist, dadurch gekennzeichnet, dass ein klares optisches Kunststoffteil mit mindestens einer Ausnehmung (19) zur Aufnahme und Befestigung mindestens eines Lichtleiterfaserbündels und/oder mindestens eines Bildleiterfaserbündels versehen ist und mit mindestens einer der oder jeder Ausnehmung und dem oder jedem darin befindlichen Faserbündel zugeordneten Linse (22), wobei Linse und Kopf ein einstückiges Teil bilden.

2. Objektivkopf nach Anspruch 1, dadurch gekennzeichnet, dass im Kopf mindestens ein Hilfsauslass (10) vorgesehen ist, der mit einem Hilfskanal der Faseroptik in Verbindung steht.

3. Objektivkopf nach Anspruch 2, dadurch gekennzeichnet, dass ein Hilfskanalauslass eine Bohrung (18) bildet, die an einem Ende offen und am gegenüberliegenden Ende im wesentlichen durch einen Vorsprung (22) geschlossen ist, der ein Teil des Kopfes ist und der an einer vorbestimmten Stelle einen Durchlass (24) aufweist und Flüssigkeit durch die Bohrung auf die Vorderseite des Kopfes geleitet ist.

4. Objektivkopf nach Anspruch 1 bis 3, dadurch gekennzeichnet, dass der Kopf aus einem Polycarbonatharz gegossen ist.

5. Objektivkopf nach Anspruch 1 bis 4, dadurch gekennzeichnet, dass der Kopf eine äussere Beschichtung aus einem kratzfesten durchsichtigen Material aufweist.

6. Bildübertragende Faseroptik für medizinische, veterinärmedizinische und industrielle Anwendungen, welche am distalen Ende bzw. am Objektivende einen Objektivkopf aufweist, der mit Ausnehmungen zur Aufnahme von Bild- und Lichtleiterfaserbündeln sowie mit Durchlässen für Hilfskanäle für Luft, Befeuchtung, Saug- und Biopsieinstrumenten versehen ist, gekennzeichnet durch einen Objektivkopf gemäss den Ansprüchen 1 bis 5.

7. Verfahren zur Herstellung eines Objektivkopfes für eine Faseroptik mit mindestens einer Ausnehmung zur Aufnahme eines Faserbündels gekennzeichnet durch folgende Schritte:

Herstellung einer Gussform in der gewünschten Form des Kopfes,

Einsetzen eines Kerns in die Gussform, so dass eine Bodenwand der Ausnehmung linsenförmig ausgebildet ist,

Giessen des Kopfes mit einer optisch klaren Kunststoffmasse und

Entfernen des Kerns aus dem gegossenen Kopf, so dass ein gegossener Objektivkopf mit der Linse entsteht und beides ein einteiliges Stück bildet.

8. Verfahren nach Anspruch 7, dadurch gekennzeichnet, dass zusätzlich die Aussenseite des Kopfes mit einer Haut oder einem Film aus optisch durchsichtigem kratzfestem Material beschichtet wird.

## Revendications

1. Tête porte-objectif pour un fibroscope transmetteur d'images pour applications médicales, vétérinaires et industrielles, du type ayant, à son extrémité éloignée portant un objectif, une tête porte-objectif ayant des cavités pour recevoir des faisceaux de fibres obtiques de transmission d'image et d'éclairage, ainsi que des orifices pour des conduits de service tels que pour l'insufflation d'air, l'irrigation, l'aspiration et pour des instruments de biopsie, caractérisée en ce qu'elle comprend une structure en plastique optiquement transparent ayant au moins une cavité (19) pour recevoir et ancrer au moins un faisceau de fibres optiques formant un guide de lumière et/ou au moins un faisceau de fibres optiques de transmission d'images, avec au moins une lentille (22) associée à la cavité ou aux cavités ainsi qu'au faisceau ou à chaque faisceau de fibres optiques contenu dans ladite cavité ou lesdites cavités, les lentilles et la tête constituant une structure intégrale d'une seule pièce.

2. Tête porte-objectif selon la revendication 1, dans laquelle la tête est formée avec au moins un orifice de service (16) utilisable pour communiquer avec un conduit de service dans un fibroscope.

3. Tête porte-objectif selon la revendication 2, dans laquelle un orifice de conduit de service délimite un alésage (18) ouvert à une extrémité et pratiquement fermé à l'extrémité opposée par une protubérance (23) formant partie intégrale de la tête, ladite protubérance ayant un orifice (24) situé en une position prédéterminée pour diriger un fluide traversant ledit alésage en travers de la face de ladite tête.

4. Tête porte-objectif selon l'une quelconque des revendications 1 à 3, moulée en résine de polycarbonate.

5. Tête porte-objectif selon l'une quelconque des revendications 1 à 4 ayant un revêtement extérieur en matière optiquement transparente et résistante aux rayures.

6. Fibroscope transmetteur d'images pour applications médicales, vétérinaires et industrielles, du type ayant à son extrémité éloignée portant un objectif, une tête porte-objectif ayant des cavités pour recevoir des faisceaux de fibres optiques de transmission d'images et d'éclairage, ainsi que des orifices pour des conduits de service tels que pour l'insufflation d'air, l'irrigation, l'aspiration, et pour des instruments de biopsie, caractérisé en ce qu'il comporte une tête porte-objectif telle que revendiquée dans l'une quelconque des revendicatons 1 à 5.

7. Procédé de fabrication d'une tête porte-objectif de fibroscope, ladite tête ayant au moins une cavité pour recevoir un faisceau de fibres optiques, comportant les opérations qui consistent:

à prévoir un moule dans la configuration voulue pour la tête,

à placer un noyau dans le moule de telle sorte qu'une paroi du fond de ladite cavité délimite une forme de lentille,

à mouler la tête en utilisant une matière plastique optiquement transparente, et

à extraire le noyau du moule de manière à créer une tête porte-objectif moulée en plastique, la lentille et la tête formant une structure intégrale d'une seule pièce.

8. Procédé selon la revendication 7, comportant l'opération supplémentaire qui consiste à revêtir l'extérieur de la tête d'une pellicule ou d'un film de matière transparente de qualité optique et résistance aux rayures.

**FIG. I**

PRIOR ART

**FIG. 2**

**FIG. 3**

**FIG. 4**